Europäisches Patentamt

European Patent Office

Office européen des brevets

(11) Publication number: **0 216 080 B1**

(12) **EUROPEAN PATENT SPECIFICATION**

(45) Date of publication of patent specification: **03.03.93**

(51) Int. Cl.5: **C12N 15/70**, C12P 21/02, C12N 9/24, C12N 9/42, C12N 1/20, //(C12N1/20, C12R1:19)

(21) Application number: **86110534.4**

(22) Date of filing: **30.07.86**

The file contains technical information submitted after the application was filed and not included in this specification

(54) **Novel plasmids and transformants.**

(30) Priority: **30.07.85 JP 168288/85**

(43) Date of publication of application:
**01.04.87 Bulletin 87/14**

(45) Publication of the grant of the patent:
**03.03.93 Bulletin 93/09**

(84) Designated Contracting States:
**DE FR NL SE**

(56) References cited:
**EP-A- 0 121 138**
**EP-A- 0 187 382**

**CHEMICAL ABSTRACTS, vol. 86, no. 19, 9th May 1977, page 264, abstract no. 136179y, Columbus, Ohio, US; R. RODRIGUEZ et al.: "Construction and characterization of cloning vehicles", & ICN-UCLA SYMP. MOL. CELL. BIOL. 1976, 5 (MOL. MECH. CONTROL GENE EXPRESSION), 471-7**

(73) Proprietor: **RIKAGAKU KENKYUSHO**
**2-1 Hirosawa**
**Wako-shi Saitama-ken(JP)**

(72) Inventor: **Horikoshi, Koki**
**No. 39-8, Sakuradai, 4-chome**
**Nerima-ku Tokyo(JP)**
Inventor: **Kudo, Toshiaki**
**No. 21-20-606, Tairamachi 1-chome**
**Meguro-ku Tokyo(JP)**
Inventor: **Kato, Chiaki**
**No. 1607-2, Nisshincho 2-chome**
**Oomiya-shi Saitama-ken(JP)**
Inventor: **Kobayashi, Tetsuo**
**50B Cedar Lane**
**Highland Park New Jersey 08904(US)**

(74) Representative: **Vossius & Partner**
**Siebertstrasse 4 P.O. Box 86 07 67**
**W-8000 München 86 (DE)**

JOURNAL OF BACTERIOLOGY, vol. 153, no. 3, March 1983, pages 1479-1485, American Society of Microbiology; J.F. SABIK et al.: "CEA-KIL operon of the ColE1 plasmid"

CHEMICAL ABSTRACTS, vol. 101, no. 3, 16th July 1984, abstract no. 18470s, Columbus, Ohio, US; N. SASHIHARA et al.: "Molecular cloning and expression of cellulase genes of alkalophilic Bacillus sp. strain N-4 in Escherichia coli", & J. BACTERIOL, 1984, 158(2), 503-6

CHEMICAL ABSTRACTS, vol. 105, no. 9, 1st September 1986, page 154, abstract no. 73432p, Columbus, Ohio, US; T. KOBAYASHI et al.: "Excretion of the penicillinase of an alkalophilic Bacillus sp. through the Escherichia coli outer membrane is caused by insertional activation of the kil gene in plasmid pMB9", & J. BACTERIOL. 1986, 166(3), 728-32

CHEMICAL ABSTRACTS, vol. 105, no. 17, 27th October 1986, page 198, abstract no. 147499q, Columbus, Ohio, US; C. KATO et al.: "Construction of an excretion vector: extracellular production of Aeromonas xylanase and Bacillus cellulases by Escherichia coli", & FEMS MICROBIOL. LETT. 1986, 36(1), 31-4

CHEMICAL ABSTRACTS, vol. 107, no. 5, 3rd August 1987, page 199, abstract no. 34339k, Columbus, Ohio, US; & JP-A-62 29 982 (INSTITUTE OF PHYSICAL AND CHEMICAL RESEARCH) 07-02-1987

**Description**

Field of the Invention

The present invention relates to a novel plasmid and a novel microorganism transformed therewith.

Background of the Invention

Plasmids are extrachromosomal genes of cyclic DNA found in microorganism cells. Plasmids are currently being used as a means of genetic recombination of microorganism and becoming more and more important in the field of research on fermentation industry.

Studies have recently been made on plasmids containing foreign DNA coding for metabolic products or particular requisites for growth of microorganism, as seen in production of amino acids or peptides. Some plasmids have been introduced into host microorganisms to obtain transformants.

The present inventors succeeded in constructing a novel plasmid into which DNA coding for extracellular production (secretion) of penicillinase was inserted. This DNA was prepared from chromosomal DNA of a microorganism belonging to the genus Bacillus. The present inventors, further, succeeded in obtaining a novel and useful transformant, Escherichia coli HB101 (pEAP2), by introducing the above plasmid into the strain Escherichia coli HB101 (Japanese Patent Publication (unexamined) 162886/1984).

Further, the present inventors succeeded in constructing a novel plasmid into which DNA coding for extracellular production (secretion) of xylanase was inserted. This DNA was prepared from chromosomal DNA of a microorganism belonging to the genus Bacillus, and then obtained a novel and useful transformant, Escherichia coli HB101 (pCX 311), by introducing the above plasmid into the strain Escherichia coli HB101 (Japanese Patent Publication (unexamined) 126085/1985).

Then, the present inventors succeeded in constructing a novel plasmid by inserting a DNA fragment coding for production of a desired useful, physiologically active substance into a plasmid into which DNA coding for extracellular production of penicillinase had been inserted and, further, succeeded in obtaining a novel and useful transformant, Escherichia coli HB101 (pXP102-3), by introducing the above plasmid into Escherichia coli HB 101 (Japanese Patent Application 278681/1984).

Methods as mentioned above are very useful where a plasmid having a DNA fragment coding for the production and extracellular secretion of a useful, physiologically active substance, i.e., ability of producing a useful, physiologically active substance, secreting it outside a cell and accumulating it, is introduced into a host to transform it and the resultant transformant is cultured to secrete the useful, physiologically active substance outside the cell, which is then isolated and collected. The reasons for the above usefulness of such methods are that destruction of the cell is unnecessary; purification of the useful, physiologically active substance is easy; and the substance is not accumulated inside the cell, so that the production of the substance is not saturated.

However, it is usual that physiologically active substances are accumulated inside a cell and only a limited number of microorganisms are known to secrete desirable, useful, physiologically active substances outside their cells. Accordingly, if it is possible to create such microorganisms as producing and extracellularly secreting desired, useful, physiologically active substances, this will have great utility in industry.

Escherichia coli, which has widely been used as a host in the recombinant DNA field and which has been studied genetically and biologically in detail, does not or hardly secrete its products outside the cells. The same properties are exhibited by transformants of Escherichia coli.

In direct contrast, Bacillus subtilis which is one of the typical bacteria of the genus Bacillus, produces and secretes enzymes, but it is genetically unstable, i.e. its genotype has a tendency to change. Further, its products are often decomposed by a protease produced by the microorganism itself.

It should also be noted that the recombinant DNA technology has been applied to yeasts but this technology has not yet achieved such a status that it is suitable for practical application.

Unfortunately, there has been a need in the art of new transformants of Escherichia coli which are capable of secreting a useful product.

The present inventors have made researches to see which gene plays a role in the extracellular secretion and have determined a DNA region which is capable of inducing the extracellular secretion of useful, physiologically active substances in transformed host and a promoter DNA region which regulates expression of the former DNA region. Further, the inventors have succeeded in constructing a plasmid having these DNA regions and have completed the present invention.

Summary of the Invention

According to one aspect of the present invention, there is provided a plasmid having a DNA region which is capable of inducing the extracellular secretion of a useful, physiologically active substance in a transformed host and a promoter DNA region which regulates the expression of the former DNA region.

According to another aspect of the invention, there is provided a plasmid which is constructed by inserting a DNA fragment coding for a particular useful, physiologically active substance into the above plasmid.

According to further aspect of the invention, there is provided a microorganism which is transformed with this plasmid.

According to still further aspect of the invention, there is provided a process for the production of a useful, physiologically active substance by culturing the above transformant.

Brief Description of the Accompanying Figures

Fig. 1 shows procedure of constructing plasmid pEAP7ΔP and a restriction endonuclease cleavage map.

Fig. 2 shows the base sequence and amino acid sequence of the "kil" gene.

Fig. 3 shows the base sequence of Ex promoter region, wherein S.D. sequence means Shine-Dalgano sequence.

Fig. 4 shows a brief depiction of insertion of xylanase DNA (xylL) or cellulase DNA (cel N or cel F) into vector plasmid pEAP7ΔP.

Detailed Description of the Invention

"Useful, physiologically active substance" used herein means a high-molecular, physiologically active substance, for instance, enzymes such as penicillinase, xylanase, β-galactosidase, β-lactamase, alkaline phosphatase and cellulase, peptide type hormones such as insulin, human growth hormone, somatostatin, secretin and endorphins, and immunologically active substances such as interferons and thymosin.

According to a preferred embodiment of the invention, provided is a plasmid which has a DNA region derived from plasmid pMB9, comprising the K-gene, as a DNA region which is capable of inducing the extracellular secretion of useful, physiologically active substance in a transformed host, and also has a DNA region derived from chromosomal DNA of Bacillus sp. No. 170 FERM-BP-467 as a promoter DNA region which regulates expression of the former DNA region.

According to a more preferred embodiment, a plasmid is provided wherein the above DNA region derived from plasmid pMB9 is the "kil" gene (hereinafter referred to as "K gene") and the DNA region derived from the chromosomal DNA of Bacillus sp. No. 170 is a DNA region restricted by restriction enzymes, Hind III - Hinc II. As a typical example of such a plasmid mentioned is plasmid pEAP7ΔP.

Plasmid pMB9, derived from pMB1 (see Bolivar et al., Gene 2:75-93 (1977)), which contains the K gene (Sabik et al., J. Bacteriolozy 153:1479-1485 (1983)) and which is responsible for colicin El release by cell lysis. However, the gene cannot be expressed in pMB9 because the plasmid lacks both the gene for colicin El production and its promoter, which are both necessary for K gene expression. K gene is similar to Col El. Sabik et al. also reported that K gene of Col El is required for mitomycin-induced lethality and release of colicin.

Construction of plasmid pEAP7ΔP

Plasmid pEAP7ΔP may be obtained from, for instance, plasmid pEAP1 as mentioned below.

Plasmid pEAP1 is a plasmid which was previously constructed by the present inventors and may be obtained as follows.

Construction of plasmid pEAP1

Chromosomal DNA of Bacillus sp. No. 170 (FERM BP-467) coding for penicillinase is cleaved by a restriction enzyme, EcoRI, and these DNA fragments are inserted into an EcoRI site of a vector plasmid, pMB9, by a shotgun method, which is then introduced into Escherichia coli HB101 and screened for resistance to ampicillin (Ap) and tetracycline (Tc). Plasmid pEAP1 is isolated from the resultant transformants (Journal of Bacteriology, Nov. 1983, p 949 - 951).

4

Plasmid pEAP3

During passage of Escherichia coli HB101 harboring plasmid pEAP1, a mutant having an enhanced penicillinase activity (three times as high as that of plasmid pEAP1) (Ap$^r$ • Tc$^s$) is obtained. A plasmid is prepared from the mutant according to a conventional technique (F. Bolivar et al., Gene, 2, 95 (1977)). Thus, plasmid pEAP3 is obtained which lacks about 4 kb upstream of structural gene of penicillinase (pen).

Construction of plasmid pEAP6

Plasmid pEAP3 is cleaved by restriction enzymes, EcoRI and Hind III and treated by Escherichia coli DNA polymerase I (Klenow fragment) (Maniatis et al., Molecular Cloning, A Laboratory Manual, CSH, 1982, p 113) to change the cleavage face to a flush end and ligated by DNA ligase T4. The resultant DNA is introduced into Escherichia coli HB101 to transform it by, for instance, a CaCl$_2$ treatment method (E. M. Lederberg and S. N. Cohen, J. Bacteriol., 119, 1072 (1974)). A plasmid is prepared from an Ap$^r$ transformant by a similar technique as mentioned above to obtain plasmid pEAP6 which lacks an EcoRI - Hind III DNA fragment of about 1 kb.

Construction of plasmid pEAP7

In order to obtain a chloramphenicol resistant (Cm$^r$) gene as a genetic marker, plasmid pBR329 (Louis Covarrubias and Francisco Bolivar, Gene, 17, 79-89 (1982)) is digested by restriction enzyme Acc II and an Acc II DNA fragment of 1.3 kb is collected. Then, pEAP6 is digested by restriction enzyme Sma I and mixed with the above Acc II DNA fragment and subjected to a coupling reaction by DNA ligase T4. The plasmid obtained is then introduced into Escherichia coli HB101 to transform it in a similar way as mentioned above. A chloramphenicol and ampicillin resistant transformant is separated and a plasmid is prepared by a conventional technique as mentioned above to obtain plasmid pEAP7.

Construction of plasmid pEAP7ΔP

Plasmid pEAP7 is digested by Hinc II to delete a Hinc II DNA fragment of 2.3 kb and subjected to a coupling reaction by DNA ligase T4, which is then introduced into Escherichia coli HB101 to transform it in a similar way as mentioned above. Then, an ampicillin sensitive strain is separated from chloramphenicol resistant (Cm$^r$) transformants, and a plasmid is prepared by a conventional technique as mentioned above to obtain plasmid pEAP7ΔP.

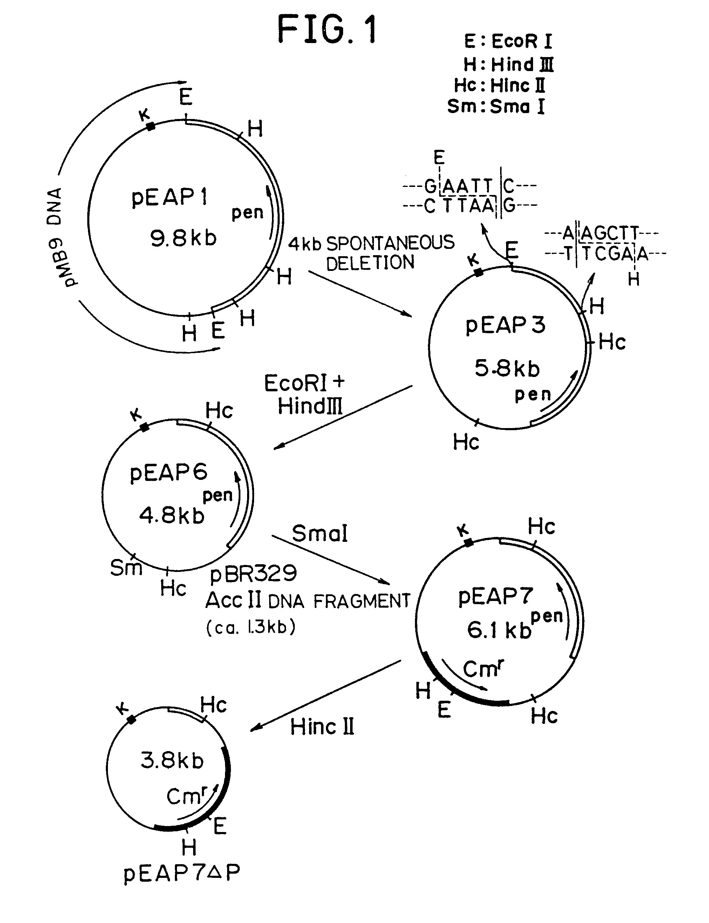

The above procedure is shown in Fig. 1.

Plasmid pEAP7ΔP thus obtained is a cyclic DNA molecule of 3.8 kb which contains a DNA region which is capable of inducing extracellular secretion of useful, physiologically active substances in transformed host, designated K gene; a promoter DNA gene which regulates expression of K gene (Hind III - Hinc II DNA region, about 0.3 kb, designated Ex promoter); and a Cm$^r$ DNA as a genetic marker. It has only one Hinc II site.

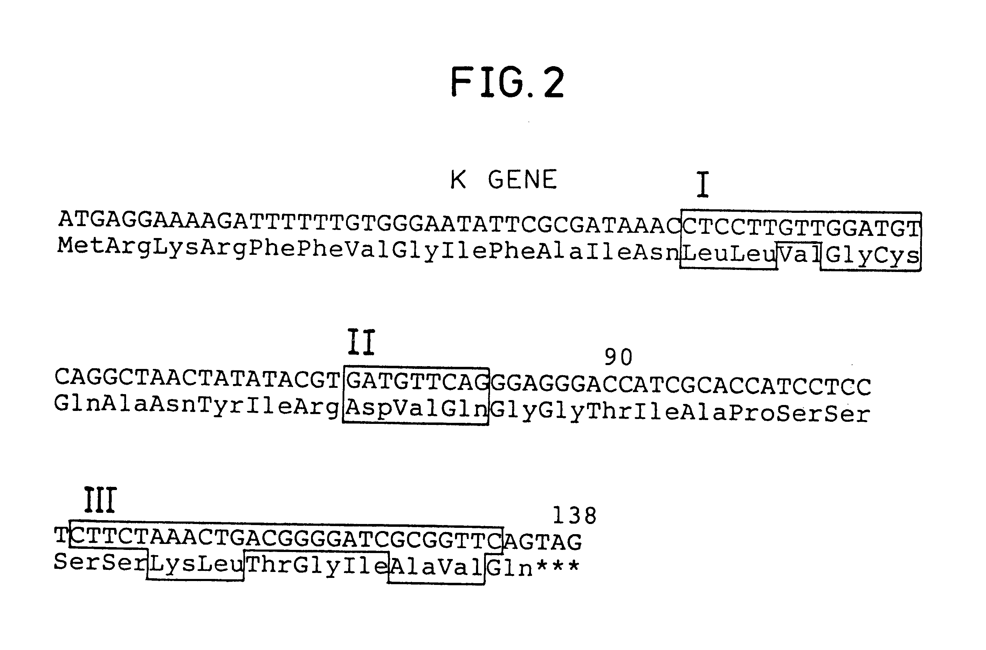

Fig. 2 shows the base sequence and amino acid sequence of K gene.

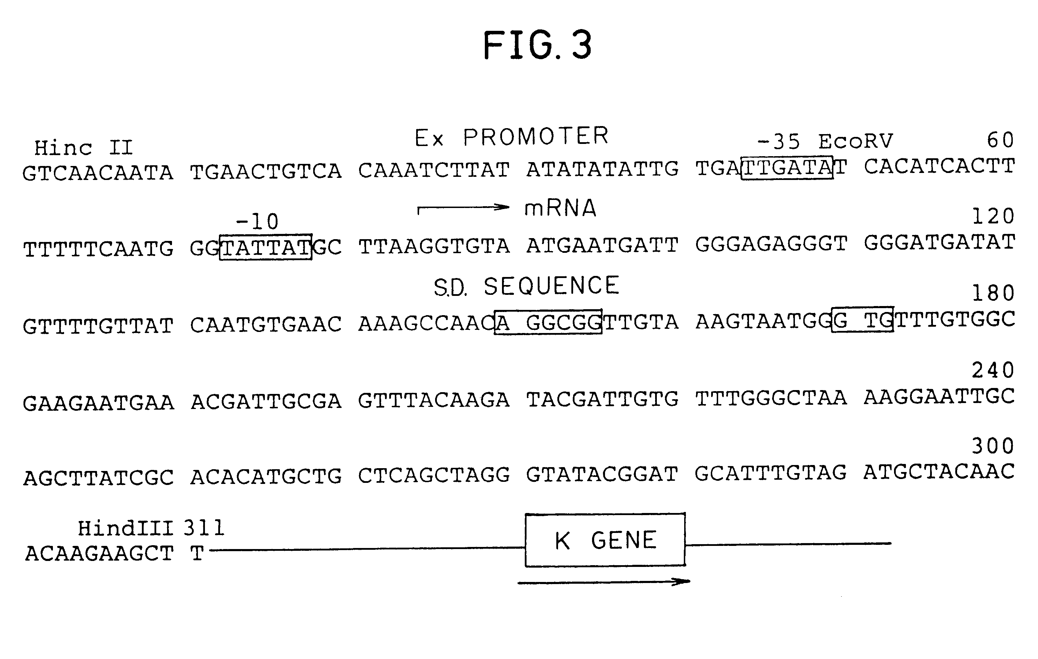

Fig. 3 shows the base sequence of Ex promoter.

Mechanism of the extracellular secretion is believed to be that K gene and Ex promoter make the outer membrane of Escherichia coli more permeable, i.e., leaky, and polymeric substances present in a periplasmic space are accordingly discharged.

For the DNA regions of K gene and Ex promoter, DNA regions which are equivalent to these in terms of biological functions, i.e., DNA regions related with these DNA regions by replacement of nucleotide, deletion of nucleotide, insertion of nucleotide, inversion of nucleotide sequence or other mutation, may of course be used.

Plasmid pEAP7ΔP may be introduced into a proper host to transform it and be replicated by proliferation of the transformant. Microorganisms which are usually used in this sort of technology may preferably be used as the host, for instance, microorganisms belonging to the genus Escherichia such as the aforesaid Escherichia coli HB101, C600, DP, supF, χ1776 and LE392. Representative of such is Escherichia coli HB101 (D. S. Goldfarb etal. Proc. Natl. Acad. Sci. U.S.A.), 79, 5886 (1982), inheritance: pro, euB, Bl, ℓacY, hsdR, hsdM, aral4, gaℓkz, xyℓ5, mtℓl, supE44, F$^-$, endoI$^-$, recA $^-$, str).

The microorganism obtained by introducing the aforesaid plasmid pEAP7ΔP into Escherichia coli HB101 is a novel microorganism, designated Escherichia coli HB101 (pEAP7ΔP) and deposited with the Fermentation Research Institute, Agency of Industrial Science and Technology, Ministry of International Trade and Industry, Japan, International Depository Authority (hereinafter referred to as "FERM") under accession number FERM BP-1101 on July 29, 1985. Microbiological properties of Escherichia coli HB101 (pEPA7ΔP) thus obtained are the same as those of Escherichia coli HB101 which is a DNA recipient.

The resultant plasmid, pEAP7ΔP, is used as a vector plasmid into which the aforesaid DNA fragment coding for a useful, physiologically active substance is inserted, so that a plasmid which is capable of inducing extracellular secretion of a particular useful, physiologically active substance in transformed host is obtained.

Representative examples of such plasmids are plasmid p7AX2 in which xylanase DNA (xyl L) derived from a chromosomal DNA of Aeromonas sp. No. 212 has been inserted, plasmid p7NKl in which cellulase DNA (cel N) derived from a chromosomal DNA of Bacillus sp. No. N-4 has been inserted, and plasmid p7FKl in which cellulase DNA (cel F) derived from a chromosomal DNA of Bacillus sp. No. 1139 has been inserted.

Construction of plasmid p7AX2

Plasmid pAX1 containing a DNA fragment coding for xylanase derived from a chromosomal DNA of Aeromonas sp. No. 212 (ATCC 31085) (Japanese Patent Application 278681/1984) is cleaved by restriction enzyme Bgl II, then treated by Escherichia coli DNA polymerase I (Klenow fragment) to change the cleavage face to a flush end and poured onto 1% agarose gel, which is then subjected to electro-elution to isolate a DNA fragment of 4.0 kb containing xyl L DNA.

The aforesaid vector plasmid, pEAP7ΔP, is cleaved by Hinc II, to which the above xyl L DNA fragment of 4.0 kb is added and ligated by DNA ligase T4. The resultant plasmid is introduced into a host by a conventional technique to transform it. A transformant having resistance to chloramphenicol and xylanase activity is separated, from which plasmid is prepared to obtain plasmid p7AX2 containing xyl L DNA of about 4 kb.

Construction of plasmid p7NK1

Plasmid pNK1 containing a DNA fragment coding for cellulase derived from chromosomal DNA of Bacillus sp. No. N-4 (ATCC 21833) (J. Bacteriol., 158, 503 - 506 (1984)) is digested by Hind III and treated in a similar way as in the case of plasmid p7AX2 to isolate a DNA fragment of 2.0 kb containing cel N DNA.

Vector plasmid pEAP7ΔP is digested by Hinc II, to which the aforesaid cel N DNA fragment of 2.0 kb is added and ligated by DNA ligase T4. The resultant plasmid is introduced into a host by a conventional technique to transform it. A transformant having resistance to chloramphenicol and cellulase activity is separated, from which plasmid is prepared to obtain plasmid p7NK1 containing cel N DNA of about 2.0 kb.

Construction of plasmid p7FK1

Cel F DNA - containing plasmid pFK1

Chromosomal DNA of Bacillus sp. No. 1139 (Nippon Nogei Kagakukai, 1985 Annual Meeting (Sapporo), Summary, p.32 1B - 28, p.707 2V - 19) is cleaved by Hind III. The DNA fragment is inserted into the Hind III site of vector plasmid pBR322 by a shotgun method, which is then introduced into Escherichia coli HB101 and a transformant having resistance to Ampicillin and cellulase activity is screened, from which plasmid pFK1 is obtained by a conventional method.

Plasmid pFK1 contains cel F DNA of 4.6 kb and in resistant to ampicillin.

The above plasmid pFK1 in digested by Hind III and Hinc II and treated in a similar way as in the case of plasmid p7AX2 to isolate a DNA fragment of 2.8 kb containing cel F DNA.

Vector plasmid pEAP7ΔP is cleaved by Hinc II, to which the aforesaid cel F DNA fragment of 2.9 kb is then added and ligated by DNA ligase T4. The resultant plasmid is introduced into a host by a conventional technique to transform it. A transformant having resistance to chloramphenicol and cellulase activity is separated, from which plasmid is prepared to obtain plasmid p7FK1 containing cel F DNA of about 2.9 kb.

The resultant plasmid which is capable of inducing extracellular secretion of a useful, physiologically active substance in a transformed host may be introduced into a proper host by conventional techniques to obtain a transformant. The useful, physiologically active substance may extracellularly be obtained in a remarkable amount by culturing the transformant. Microorganisms belonging to the genus Escherichia may be used as hosts. Microorganisms which are usually used in this kind of technology, such as the aforesaid Escherichia coli HB101, C600, DP, supF, X 1776 and LE392, may be preferably used. Typically used is Escherichia coli HB101.

Microorganisms obtained by introducing the aforesaid plasmid p7AX2, p7NK1 or p7FX1 are novel and designated Escherichia coli HB101 (p7AX2), Escherichia coli HB101 (p7NK1), Escherichia coli HB101 (p7FK1), respectively, which are deposited with FERM on July 29, 1985 under accession number FERM BP-1102, FERM BP-1103 and FERM BP-1104, respectively.

The Assignee, Rikagaku Kenkyusho, commits itself to maintain the FERM BP-cultures for a period of thirty years from the filing date of the present application, that is up to the year 2015 or for a period of five years from the date of the last request.

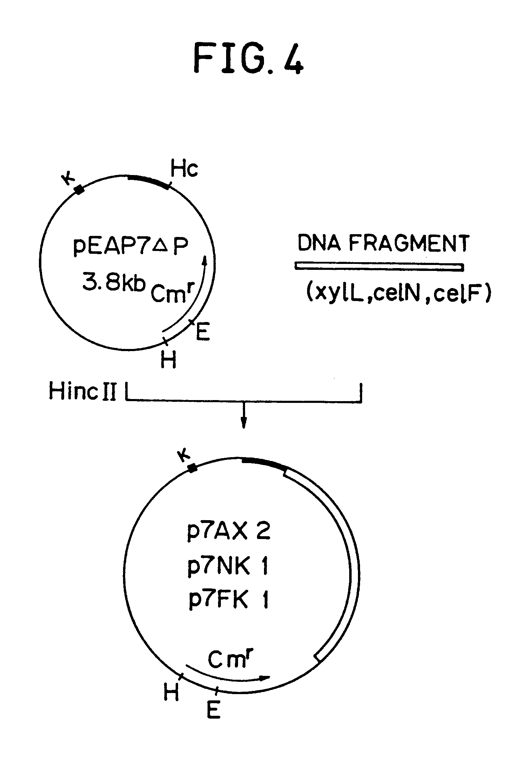

Microbiological properties of each microorganism thus obtained are the same as those of Escherichia coli HB101 which is a DNA recipient. The above procedure is shown in Fig. 4.

Production of useful, physiologically active substance

For culturing the transformants obtained in the above procedure, a culture medium proper to production of substances on a basis of particular genetic information and proper to growth of the host microorganism may be used. In the method according to the invention, a LB broth (trypton, yeast extract NaCl), a BPB medium (Difco; polypeptone, yeast extract, potassium phosphate), a nutrient • agar medium (Difco 0001) and trypton - sodium chloride medium, all of which are usually used as a growth medium for Escherichia coli, may be prepared as a basis medium and used.

In addition, nutrients such as amino acids and vitamins other than carbon sources and nitrogen sources may be added if desired.

In a culturing method, conditions of pH, temperature, amount of supplied oxygen, etc., may be selected so as to be proper to growth of microorganisms of the genus Escherichia. However, after the aforesaid microorganism is inoculated to the medium, it is preferred to continue culturing in one and the same medium in a period of time after the microorganism grows and the amount of cells reaches maximum, that is to say a late logarithmic growth phase, until production and accumulation of a high molecular substance in the medium cease substantially.

The period of time after the amount of cells of the aforesaid microorganism of Escherichia coli reaches maximum until the production and accumulation of the high molecular substances in the medium cease substantially is in a range from about 12 to about 48 hours. A pH condition are not particularly influential. However, a pH of from 5 to 8, particularly of 7, is proper.

In the present invention, most of the physiologically active substance produced by the transformant is secreted outside the cells and found in the culture medium which is filtrated to remove the cells. The resultant filtrate is then treated according to a conventional isolation and purification method for this kind of useful, physiologically active substance, such as ammonium sulfate fractionation, lyophilization, etc.

On the other hand, the resultant cells are collected and extracted by centrifugation, sonication, etc., and then treated according to the same conventional isolation and purification method as described above to obtain a minor portion of the products.

The present invention will further be explained by unrestrictive reference examples and working examples.

Reference Example (Preparation of plasmid pEAP1)

(1) Preparation of chromosomal DNA coding for penicillinase production.

Alkalophilic Bacillus No. 170 (FERM BP-467), which has ability of production and extracellular accumulation of penicilinase was cultured in a medium (glycerol 2.0 g/ℓ, yeast extract 5.0 g/ℓ, polypeptone 5.0 g/ℓ, $K_2HPO_4$ 1.0 g/ℓ, $MgSO_4 \cdot 7H_2O$ 0.2 g/ℓ, pH 9.0 by $NaHCO_3$ 10 g/ℓ) at a temperature of 30°C for 19 hours with shaking, and cells after a late logarithmic growth phase were collected. Chromosomal DNA was extracted by DNA extraction according to a phenol method and purified to yield 5 mg of chromosomal DNA.

(2) Insertion of chromosomal DNA fragment into vector

To 10 μg of the chromosomal DNA obtained in (1) above, there was added restriction endonuclease EcoRI and incubated at 37°C to cleave the former. Tetracycline resistant (Tet$^r$) plasmid pMB9 (Bethesda Research Laboratories, USA) to be used as a vector was completely cleaved by EcoRI and heat treated at 65°C for 5 minutes, which was then mixed with the aforesaid DNA fragments, subjected to ligation reaction of DNA chains by a DNA ligase derived from T4 phage at 10°C for 24 hours and heat treated at 65°C for 5

minutes. Subsequently, two times volume of ethanol was added to the reaction solution to precipitate plasmid DNA in which the chromosomal DNA was inserted, which was recovered.

(3) Transformation by plasmid pEAP1

Strain Escherichia coli HB101 (Molecular Cloning A Laboratory Manual P504 (1982)) (inheritance: $F^-$, hsdS20 ($r^-B$, $m^-B$), rec Al3, ara$^-$ 14, pro A2, lac Yl, galK2, rps L20 (Sm$^1$), xyl-5, mtl-1, sup E44λ$^-$), which is a hybrid strain of strain Escherichia coli K-12 and strain Escherichia coli B, was inoculated to 10 mℓ of LB broth (tripton (Difco) 10 g/ℓ, yeast extract 5 g/ℓ, glucose 1 g/ℓ, NaCℓ 10 g/ℓ, pH 7.0) and incubated at 37°C with shaking to grow until a late logarithmic growth phase and, then, collected. This was suspended in a CaCℓ$_2$ solution of a final concentration of 0.03M under ice cooling to prepare competent cell. To this cell suspension there was added the plasmid DNA solution obtained in (2) above and reacted under ice cooling for 60 minutes and given heat shock at 42°C for 1 to 2 minutes to incorporate the above plasmid DNA into the cells.

Among these transformed strains, a strain resistant to ampicillin 20 μg/mℓ and tetracyclin 50 μg/mℓ was screened. A cell suspension of the above resistant strain was inoculated to LB broth as mentioned above and incubated at 37°C for 3 to 5 hours with shaking. Then, the cells were collected and washed to yield Escherichia coli HB101 (pEAP1) which was transformed by plasmid pEAP1.

These cells were treated as follows to obtain purified plasmid pEAP1:

Cells

    10 mℓ, 20% Sucrose, 50 mM Tris, 1 mM EDTA pH 8,

    Suspended, Keep on ice

50 mℓ polypropylene centrifuge tube.

    2 mℓ, 0.25M EDTA.

    1 mℓ, Lysozyme (5 mg/mℓ of 0.025 M Tris, pH 8)

    0.1 mℓ, RNase (10 mg/mℓ)

Cell lysis. Mix gently. Stand on ice for 15 to 30 min.

    5 mℓ, 3x Triton. Mix gently. Stand on ice for 15 to 45

    min.

Centrifugation. 17,000 rpm, 40 min. at 4°C.

Supernatant

250 mℓ Glass bottle.

    2/3 vol. DD $H_2O$

    2/3 vol. Cold saturated phenol. Mix gently

Centrifugation. 6,500 rpm, 15 min. at 4°C.

Upper layer.

    Equal vol. of phenol; chloroform.

Centrifugation. 6,500 rpm, 15 min. at 4°C.

Upper layer.

    1/25 vol. 5 M NaCl.

    2 vol. EtOH. Over night at -20°C.

Centrifugation. 6,500 rpm, 60 min. at -20°C.

DNA pellets. Dry excess liquid.

    5 ml A-50 buffer, resuspended.

    1 ml sterile 80% glycerol, mix gentry.

A-50 column. (2 x 35 cm, 1 fraction = 4 m .)

DNA fraction. (A$_{260}$ peak.)

    2 vol. EtOH. Over night at -20°C.

Centrifugation. 6,500 rpm, 60 min. at -20°C.

DNA pellets.

    2.1 ml TEN buffer in 5 m  cellulose nitrate tube.

    2.2 g CsCl, mix.

    (In dark.)

    150 μl PdI (2 mg/ml). Mix well.

    2 ml mineral oil.

CsCl gradient centrifugation. 36,000 rpm. 40 hrs at 20°C.

                           Upper band; chromosomal, nicked

Observe with UV.   DNA.

                           Lower band; covalently bonded,

                           closed p-DNA.

Collect lower band DNA dropwise.

Dowex 50W-X8 column. Monitor with UV.

    (In light.)

Dialysis against 2 to 4 l of 10 mM Tris, 1 mM EDTA, pH 8.

                           Over night at 4°C.

In 30 ml Cortex centrifuge tube.

    1/25 vol. 5M NaCl.

    2 vol. EtOH.    Over night at -20°C.

Centrifugation. 6,500 rpm, 60 min. at -20°C.

10

↓

```
DNA precipitates.

        1 - 2 mℓ TEN buffer.

Purified plasmid DNA (1 mg/1 mℓ culture) Stored at -20 to

-70ºC.
```

Example 1 (Preparation of plasmid pEAP3)

During passage of the aforesaid strain, Escherichia coli HB101 (pEAP1), there was obtained a mutant resistant to ampicillin (Ap$^r$) and sensitive to tetracycline (Tc$^s$) which shows enhanced penicillinase activity (about three times as strong as pEAP1). Plasmid was prepared from this mutant as in Reference Example. There was obtained plasmid pEAP3 which lacked about 4 kb upstream of structural gene of penicillinase.

Example 2 (Preparation of plasmid pEAP6)

1 μg of plasmid pEAP3 obtained in Example 1 was cleaved by adding restriction enzymes EcoRI and Hind III and reacting at 37ºC for 2 hours, to which Escherichia coli DNA polymerase I (Klenow fragment) was added and reacted at room temperature for 30 minutes to change the DNA cleavage face to a flush end and, then, subjected to ligation reaction by DNA ligase T4 at room temperature for 24 hours, heat treated at 65ºC for 5 minutes. 2 Volumes of ethanol were added to precipitate and collect plasmid DNA. The resultant DNA was introduced into Escherichia coli HB101 to transform it as in Reference Example. Plasmid was isolated from an ampicillin resistant transformant and purified as in Reference Example to yield plasmid pEAP6 which lacked an EcoRI-Hind III DNA fragment of about 1.0 kb.

Example 3 (Preparation of plasmid pEAP7)

10 μg of plasmid pBR329 (Gene, 17, 79 to 89 (1982)) were cleaved by adding restriction enzyme Acc II and reacting at 37ºC for 24 hours, which was poured onto 1.5% agarose gel. 1.3 kb AccII DNA fraction was collected by an electro-elution method (P.J. Greene et al. "Methods in Molecular Biology" Vol. 7, Marcell Dekker), 1974 p.87). This DNA fragment contained a chloramphenicol acetyl transferase (CAT) gene.

1 μg of plasmid pEAP6 obtained in Example 2 was cleaved by adding restriction enzyme Sma I and reacting at 37ºC for 2 hours, which was mixed with 0.5 μg of the aforesaid 1.3 kb AccII DNA fragment and, after addition of DNA ligase T4, subjected to ligation reaction at room temperature for 24 hours and then treated as in Example 2 to collect plasmid DNA. The resultant plasmid DNA was introduced into Escherichia coli HB101 to transform it as in Reference Example. Then, a transformant resistant to chloramphenicol 50 μg/mℓ and to ampicillin 20 μg/mℓ was separated, and plasmid was isolated and purified as in Reference Example to obtain plasmid pEAP7.

Example 4 (Preparation of plasmid pEAP7ΔP and its transformant)

1 μg of plasmid pEAP7 obtained in Example 3 was cleaved by adding restriction enzyme Hinc II and reacting at 37ºC for 2 hours, which was subjected to ligation reaction of DNA chain at room temperature for 24 hours and, then, treated as in Example 2 to collect plasmid DNA. The resultant plasmid DNA was introduced into Escherichia coli HB 101 to transform it as in Reference Example. An ampicillin sensitive strain was separeted from chloramphenicol 5 μg/mℓ resistant transformants, and plasmid was isolated and purified as in Reference Example to yield plasmid pEAP7ΔP.

Further, transformant Escherichia coli HB101 (pEAP7ΔP) (FERM BP-1101) containing the above pEAP7Δp was inoculated to a LB broth and incubated at 37ºC for 3 to 5 hours for proliferation.

Example 5 (Preparation of plasmid p7AX2 and its transformant)

10 g of plasmid pAX1 were cleaved by adding restriction enzyme Bgl II and reacting at 37ºC for 2 hours, to which Escherichia coli DNA polymerase I (Klenow fragment) was added and reacted at room temperature for 2 hours to change the DNA cleavage face to a flush end. This was poured onto 1.5% agarose gel, and a xylanase DNA fraction of 4.0 kb was collected by an electro-elution method.

1 μg of plasmid pEAP7ΔP obtained in Example 4 was cleaved by adding restriction enzyme Hinc II and reacting at 37°C for 2 hours, to which 0.5 to 1 μg of the aforesaid Bgl II DNA fragment of 4.0 kb was added and subjected to ligation reaction by DNA ligase T4 at room temperature for 24 hours and treated as in Example 2, and plasmid DNA was collected. The resultant plasmid DNA was introduced into Escherichia coli HB101 to transform it as in Reference Example. Among transformants resistant to chloramphenicol 50 μg/mℓ, a strain having xylanase activity was separated, from which plasmid was isolated and purified as in Reference Example to yield plasmid p7AX2.

The above transformant, Escherichia coli HB101 (p7AX2) (FERM BP-1102) was inoculated to a LB broth and incubated at 37°C for 3 to 5 hours with shaking for proliferation.

Example 6 (Preparation of plasmid p7NK1 and its transformant)

10 μg of plasmid pNK1 were cleaved by adding restriction enzyme Hind III and reacting at 37°C for 24 hours, to which Escherichia coli DNA polymerase I (Klenow fragment) was added and reacted at room temperature for 24 hours to change the DNA cleavage face to a flush end. This was poured onto 1.5% agarose gel, and a cellulase DNA fragment of 2.0 kb was collected by an electro-elution method.

1 μg of plasmid pEAP7ΔP obtained in Example 4 was cleaved by adding restriction enzyme Hinc II and reacting at 37°C for 2 hours, to which 0.5 to 1 μg of the aforesaid cellulase DNA fragment of 2.0 kb was added and subjected to ligation reaction by DNA ligase T4 at room temperature for 24 hours and, then, treated as in Example 2 to collect plasmid DNA. The resultant plasmid DNA was introduced into Escherichia coli HB101 to transform it as in Reference Example. Among transformants resistant to chloramphenicol 50 μg/mℓ, a strain having cellulase activity was separated, from which plasmid was isolated and purified as in Reference Example to obtain plasmid p7NK1.

The above transformant, Escherichia coli HB101 (p7NK1) (FERM BP-1103) was inoculated to a LB broth and incubated at 37°C for 3 to 5 hours with shaking for proliferation.

Example 7 (Preparation of plasmid p7FK1 and its transformant)

10 μ g of plasmid pFK1 were cleaved by adding restriction enzymes Hind III and Hinc II and reacting at 37°Cfor 2 hours, to which Escherichia coli polymerase I (Klenow fragment) was added and reacted at room temperature for 2 hours to change the DNA cleavage face to a flush end. This was poured onto 1.5% agarose gel, and a cellulase DNA fragment of 2.9 kb was collected by an electro-elution method.

1 μg of plasmid pEAP7ΔP obtained in Example 4 was cleaved by adding restriction enzyme Hinc II and reacting at 37°C for 2 hours, to which 0.5 to 1 μg of the aforesaid cellulase DNA fragment of 2.9 kb was added and subjected to ligation reaction by DNA ligase T4 at room temperature for 24 hours and, then, treated as in Example 2 to collect plasmid DNA. The resulting plasmid DNA was introduced into Escherichia coli HB101 to transform it. Among transformants resistant to chloramphenicol 50 μg/mℓ, a strain having cellulase activity was separated, from which plasmid was isolated and purified as in Reference Example to obtain plasmid p7FK1.

The above transformant, Escherichia coli HB101 (p7FK1) (FERM BP-1104) was inoculated to a LB broth and incubated at 37°C for 3 to 5 hours with shaking for proliferation.

Example 8 (Production and extracellular secretion by each transformant)

Microorganism

Transformants obtained in Examples 5 to 7:

| Escherichia coli | HB101 (p7AX2) (FERM BP-1102) |
| Escherichia coli | HB101 (p7NK1) (FERM BP-1103) |
| Escherichia coli | HB101 (p7FK1) (FERM BP-1104) |

were used and examined for distribution of intracellular production, periplasmic space production and extracellular production of xylanase and cellulase. For comparison, Escherichia coli HB101 strains which were transformed by each of plasmids pAX2, pNK1, and pFK1 were used.

pAX2:   a Bgl II DNA fragment of 4.0 Kb containing xyl L, Ap$^r$, Xyl$^+$ was integrated in a Bam HI restriction site of pBR322.

pNK1:   pBR322 + Hind III DNA fragment of 2.0 Kb containing cel N, Ap$^r$, Cel$^+$.

pFK1:   pBR322 + Hind III DNA fragment of 4.6 Kb containing cel F, AP$^r$, Cel$^+$.

Medium
_____

A LB broth having the following composition was used:

10 g of trypton, 5 g of yeast extract, 1 g of glucose and 10 g of NaCl were dissolved in 1$\ell$ of distilled water, and a pH was regulated by NaOH. If necessary, 20 g of agar was added to prepare a solid medium.

Culturing Method
_____

Each of the transformants was inoculated to the above LB broth and cultured at 37°C for 24 hours in a flask of 500 m$\ell$ capacity. After collecting cells, the products were fractioned into extracellular fraction, periplasmic space fraction and intracellular fraction by an osmotic shock method (Kato et al., Eur. J. Appl. Microbiol. Biotechnol) (1983) 18:339-343).

Determination of enzyme activity
_____

Xylanase activity: To 0.05 m$\ell$ of an enzyme solution, there were added 0.1 m$\ell$ of a xylan solution (Seikagaku Kogyo Co.) and 0.1 m$\ell$ of 0.2 M tris maleate buffer of pH 8.0 and reacted at 40°C for 10 minutes, to which 1 m$\ell$ of DNS (3.5-dinitrosalicylic acid) was added and reacted at 100°C for 5 minutes. Then, 4 m$\ell$ of water were added and absorbance at 510 nm was determined. An amount of enzyme to cause reduction capacity corresponding 1 mg of xylose in one minute was designated 1 unit (U) of xylanase.

Cellulase activity: To 1 m$\ell$ of carboxymethyl cellulose (CMC) (2.0%) and 1 m$\ell$ of glycine NaC$\ell$-NaOH buffer (pH 9.0), there was added 0.5 m$\ell$ of an enzyme solution and reacted at 40°C for 20 minutes.

After completion of the reaction, reducing sugars were quantified by a 3.5-dinitro-salicylic acid (DNS) method. That is, 1 m$\ell$ of a DNS agent was added to 0.25 m$\ell$ of a reaction solution and heated at 100°C for 5 minutes for color development. After cooling, 4 m$\ell$ of distilled water was added for dilution. This was subjected to colorimetric quantitative analysis at a wave length of 500 m$\mu$.

1 unit (U) of enzyme titer is defined to produce reducing sugars corresponding to 1$\mu$ mole of dextrose in 1 minute in the above conditions.

The results are shown in Table 1

Table 1

| Plasmid | Enzyme Protein | Extracellular Enzyme Activity (U/ml) | Periplasmic Enzyme Activity (U/ml) | Intracellular Enzyme Activity (U/ml) | Total enzyme Activity (U/ml) |
|---|---|---|---|---|---|
| pAX2 | Xylanase | 23 (4.8%) | 147 (30.4%) | 315 (64.8%) | 485 |
| p7AX2 | | 135 (62.1%) | 8 (3.6%) | 74 (34.3%) | 217 |
| pNK1 | Cellulase | 12 (9.2%) | 60 (44.0%) | 64 (46.8%) | 136 |
| p7NK1 | | 282 (66.7%) | 92 (21.8%) | 49 (11.5%) | 423 |
| pFK1 | Cellulase | 7 (6.1%) | 88 (74.3%) | 23 (19.6%) | 118 |
| p7FK1 | | 93 (60.2%) | 16 (10.6%) | 45 (29.2%) | 154 |

14

Effects of the invention

According to the present invention, a plasmid containing the DNA region which is capable of inducing extracellular secretion of useful, physiologically active substances in transformed host and the promoter DNA region which regulates expression of the former DNA region is used. By inserting a DNA fragment coding for the useful, physiologically active substances into the above plasmid, a plasmid can be obtained which has ability of providing a host originally having no ability of extracellular secretion, with a function of inducing the production and secretion of the useful, physiologically active substances in the transformed host. Accordingly, it is possible by the present invention to optionally prepare a plasmid which is capable of producing and extracellularly secreting useful, physiologically active substances, such as insulin, human growth hormone, interferons and enzymes. By introducing this into a host such as Escherichia coli, it is possible to create useful microorganisms which have a function of promoting extracellular secretion of the above usesul, physiologically active substances.

**Claims**

1. A plasmid containing
   (i) a DNA region derived from plasmid pMB9 comprising the k gene and being capable of inducing extracellular secretion of a useful, physiologically active substance in a transformed host;
   (ii) the Ex promoter region derived from the chromosomal DNA of Bacillus sp. No. 170 FERM BP-467 and regulating expression of the former DNA region; and
   (iii) a genetic marker;
   (iv) but not containing any DNA fragment coding for said useful, physiologically active substance.

2. The plasmid according to claim 1, wherein said Ex promoter (ii) has the nucleotide sequence given in Fig. 3.

3. The plasmid according to any one of claims 1 or 2, which is plasmid pEAP7ΔP having the restriction map given in Fig. 1 and being deposited as E. coli HB101 (pEAP7ΔP) (FERM BP-1101).

4. The plasmid according to any one of claims 1 to 3, further comprising a DNA fragment coding for a useful, physiologically active substance other than penicillinase.

5. The plasmid according to claim 4, wherein said useful, physiologically active substance is xylanase.

6. The plasmid according to claim 4, wherein said useful, physiologically active substance is cellulase.

7. The plasmid according to claim 4 or 5, which is plasmid p7AX2 having the restriction map given in Fig. 4 and being deposited as E. coli HB101 (FERM BP-1102).

8. The plasmid according to claim 4 or 6, which is plasmid p7FK1 having the restriction map given in Fig. 4 and being deposited as E. coli HB101 (FERM BP-1104).

9. The plasmid according to claim 4 or 6, which is plasmid p7NK1 having the restriciton map given in Fig. 4 and being deposited as E. coli HB101 (FERM BP-1103).

10. Escherichia coli HB101 (pEAP7ΔP) (FERM BP-1101)

11. Escherichia coli HB101 (p7AX2) (FERM BP-1102)

12. Escherichia coli HB101 (p7NK1) (FERM BP-1103)

13. Escherichia coli HB101 (p7FK1) (FERM BP-1104)

14. A process for the production of a useful, physiologically active substance characterized in that a microorganism transformed with a plasmid according to any one of claims 4 to 9 is cultured until the production and accumulation of the useful, physiologically active substance in said medium substantially ceases, and the useful, physiologically active substance is collected from the culture.

**15.** The process according to claim 14, wherein the transformed microorganism is Escherichia coli HB101 (p7AX2) (FERM BP-1102).

**16.** The process according to claim 14, wherein the transformed microorganism is Escherichia coli HB101 (p7NK1) (FERM BP-1103).

**17.** The process according to claim 14, wherein the transformed microooorganism is Escherichia coli HB101 (p7FK1) (FERM BP-1104).

**Patentansprüche**

**1.** Plasmid, enthaltend
(i) eine DNA-Region, die aus dem das K-Gen umfassenden Plasmid pMB9 stammt und die die extrazelluläre Sekretion eines nützlichen, physiologisch aktiven Stoffes in einem transformierten Wirt induzieren kann;
(ii) die aus der chromosomalen DNA von Bacillus sp. Nr. 170 FERM BP-467 stammende Ex-Promotorregion, die die Expression der genannten DNA-Region steuert; und
(iii) einen genetischen Marker;
(iv) aber nicht enthaltend ein den nützlichen physiologisch aktiven Stoff codierendes DNA-Fragment.

**2.** Plasmid nach Anspruch 1, wobei der Ex-Promotor (ii) die in Fig. 3 angegebene Nucleotidsequenz aufweist.

**3.** Plasmid nach Anspruch 1 oder 2, das das Plasmid pEAP7ΔP mit der in Fig. 1 angegebenen Restriktionskarte ist und das als E. coli HB101 (pEAP7ΔP) (FERM BP-1101) hinterlegt ist.

**4.** Plasmid nach einem der Ansprüche 1 bis 3, zusätzlich umfassend ein DNA-Fragment, das einen nützlichen, physiologisch aktiven Stoff codiert, der nicht Penicillinase ist.

**5.** Plasmid nach Anspruch 4, wobei der nützliche, physiologisch aktive Stoff Xylanase ist.

**6.** Plasmid nach Anspruch 4, wobei der nützliche, physiologisch aktive Stoff Cellulase ist.

**7.** Plasmid nach Anspruch 4 oder 5, das das Plasmid p7AX2 mit der in Fig. 4 angegebenen Restriktionskarte ist und das als E. coli HB101 (FERM BP-1102) hinterlegt ist.

**8.** Plasmid nach Anspruch 4 oder 6, das das Plasmid p7FK1 mit der in Fig. 4 angegebenen Restriktionskarte ist und das als E. coli HB101 (FERM BP-1104) hinterlegt ist.

**9.** Plasmid nach Anspruch 4 oder 6, das das Plasmid p7NK1 mit der in Fig. 4 angegebenen Restriktionskarte ist und das als E. coli HB101 (FERM BP-1103) hinterlegt ist.

**10.** Escherichia coli HB101 (pEAP7ΔP) (FERM BP-1101).

**11.** Escherichia coli HB101 (p7AX2) (FERM BP-1102).

**12.** Escherichia coli HB101 (p7NK1) (FERM BP-1103).

**13.** Escherichia coli HB101 (p7FK1) (FERM BP-1104).

**14.** Verfahren zur Herstellung eines nützlichen, physiologisch aktiven Stoffes, dadurch gekennzeichnet, daß ein mit einem Plasmid nach einem der Ansprüche 4 bis 9 transformierter Mikroorganismus gezüchtet wird, bis die Herstellung und Ansammlung des nützlichen, physiologisch aktiven Stoffes im Medium im wesentlichen beendet ist und der nützliche, physiologisch aktive Stoff aus der Kultur gewonnen wird.

**15.** Verfahren nach Anspruch 14, wobei der transformierte Mikroorganismus Escherichia coli HB101 (p7AX2) (FERM BP-1102) ist.

**16.** Verfahren nach Anspruch 14, wobei der transformierte Mikroorganismus Escherichia coli HB101 (p7NK1) (FERM BP-1103) ist.

**17.** Verfahren nach Anspruch 14, wobei der transformierte Mikroorganismus Escherichia coli HB101 (p7FK1) (FERM BP-1104) ist.

**Revendications**

**1.** Plasmide contenant
(i) une région d'ADN dérivée du plasmide pMB9 comprenant le gène K et étant capable d'induire la sécrétion extracellulaire d'une substance utile physiologiquement active dans un hôte transformé;
(ii) la région du promoteur Ex dérivé de l'ADN chromosomique de Bacillus sp. n° 170 FERM BP-467 et qui assure la régulation de l'expression de la région d'ADN précédente; et
(iii) un marqueur génétique;
(iv) mais ne contenant aucun fragment d'ADN codant pour ladite substance utile physiologiquement active.

**2.** Plasmide selon la revendication 1, dans lequel ledit promoteur Ex (ii) a la séquence de nucléotides donnée dans la figure 3.

**3.** Plasmide selon l'une quelconque des revendications 1 ou 2, qui est le plasmide pEAP7ΔP ayant la carte de restriction donnée dans la figure 1 et qui est déposé en tant que E. coli HB101 (pEAP7ΔP) (FERM BP-1101).

**4.** Plasmide selon l'une quelconque des revendications 1 à 3, qui comprend en outre un fragment d'ADN codant pour une substance utile physiologiquement active autre qu'une pénicillinase.

**5.** Plasmide selon la revendication 4, dans lequel ladite substance utile physiologiquement active est une xylanase.

**6.** Plasmide selon la revendication 4, dans lequel ladite substance utile physiologiquement active est une cellulase.

**7.** Plasmide selon la revendication 4 ou 5, qui est le plasmide p7AX2 ayant la carte de restriction donnée dans la figure 4 et qui est déposé en tant que E. coli HB101 (FERM BP-1102).

**8.** Plasmide selon la revendication 4 ou 6, qui est le plasmide p7FK1 ayant la carte de restriction donnée dans la figure 4 et qui est déposé en tant que E. coli HB101 (FERM BP-1104).

**9.** Plasmide selon la revendication 4 ou 6, qui est le plasmide p7NK1 ayant la carte de restriction donnée dans la figure 4 et qui est déposé en tant que E. coli HB101 (FERM BP-1103).

**10.** Escherichia coli HB101 (pEAP7ΔP) (FERM BP-1101)

**11.** Escherichia coli HB101 (p7AX2) (FERM BP-1102)

**12.** Escherichia coli HB101 (p7NK1) (FERM BP-1103)

**13.** Escherichia coli HB101 (p7FK1) (FERM BP-1104)

**14.** Procédé de production d'une substance utile physiologiquement active, caractérisé en ce qu'on cultive un microorganisme transformé avec un plasmide selon l'une quelconque des revendications 4 à 9 jusqu'à ce que cesse pratiquement la production et l'accumulation de la substance utile physiologiquement active dans ledit milieu, et en ce qu'on recueille la substance utile physiologiquement active à partir de la culture.

**15.** Procédé selon la revendication 14, dans lequel le microorganisme transformé est Escherichia coli HB101 (p7AX2) (FERM BP-1102).

17

**16.** Procédé selon la revendication 14, dans lequel le microorganisme transformé est Escherichia coli HB101 (p7NK1) (FERM BP-1103).

**17.** Procédé selon la revendication 14, dans lequel le microorganisme transformé est Escherichia coli HB101 (p7FK1) (FERM BP-1104).

# FIG. 1

E : EcoR I
H : Hind III
Hc : Hinc II
Sm : Sma I

# FIG.2

K GENE

```
                                           I
ATGAGGAAAAGATTTTTTGTGGGAATATTCGCGATAAACCTCCTTGTTGGATGT
MetArgLysArgPhePheValGlyIlePheAlaIleAsnLeuLeuValGlyCys

                        II                      90
CAGGCTAACTATATACGTGATGTTCAGGGAGGGACCATCGCACCATCCTCC
GlnAlaAsnTyrIleArgAspValGlnGlyGlyThrIleAlaProSerSer

   III                                      138
TCTTCTAAACTGACGGGGATCGCGGTTCAGTAG
SerSerLysLeuThrGlyIleAlaValGln***
```

EP 0 216 080 B1

# FIG. 3

Hinc II                 Ex PROMOTER        -35 EcoRV     60

GTCAACAATA TGAACTGTCA CAAATCTTAT ATATATATTG TGA TTGATAT CACATCACTT

           -10               mRNA                       120

TTTTTCAATG GG TATTAT GC TTAAGGTGTA ATGAATGATT GGGAGAGGGT GGGATGATAT

                        S.D. SEQUENCE                180

GTTTTGTTAT CAATGTGAAC AAAGCCAAC A GGCGG TTGTA AAGTAATGG G TG TTTGTGGC

                                             240

GAAGAATGAA ACGATTGCGA GTTTACAAGA TACGATTGTG TTTGGGCTAA AAGGAATTGC

                                           300

AGCTTATCGC ACACATGCTG CTCAGCTAGG GTATACGGAT GCATTGTAG ATGCTACAAC

HindIII 311

ACAAGAAGCT T————————————— | K GENE |—————————

# FIG. 4